# EUROPÄISCHE PATENTSCHRIFT

(11) **EP 1 670 745 B1**
(45) Veröffentlichungstag und Bekanntmachung des Hinweises auf die Patenterteilung: **14.03.2012**
(21) Anmeldenummer: 04765388.6
(22) Anmeldetag: 18.09.2004
(51) Int. Cl.: C07C 205/06, C07C 201/16, C02F 1/26

(54) **Verfahren zur Herstellung von Dinitrotoluol wobei nitrokresolhaltige Extrakte als Einsatzstoff mitverwendet werden.**
Method of production of dinitrotoluene whereby extracts containing nitrocresols are also used as educt
Procédé de production de dinitrotoluène comprenant aussi des extraits contenant des nitrocrésols comme réactif

(30) Priorität: 29.09.2003 DE 10345601
(43) Veröffentlichungstag der Anmeldung: 21.06.2006
(73) Patentinhaber: BASF SE, 67056 Ludwigshafen (DE)
(72) Erfinder: BÜTTNER, Johannes, 01945 Ruhland (DE); ALLARDT, Holger, 01987 Schwarzheide (DE); TONDER, Reinhard, 01998 Klettwitz (DE); REETZ, Reiner, 01987 Schwarzheide (DE); REICHELT, Michael, 01945 Ruhland (DE)
(86) Internationale Anmeldenummer: PCT/EP2004/010497
(87) Internationale Veröffentlichungsnummer: WO 2005/037766

(56) Entgegenhaltungen:
- US-A- 4 257 986
- US-A- 4 597 875
- US-A- 4 604 214
- US-A- 4 986 917
- US-B2- 6 583 327

## Beschreibung

Die Erfindung betrifft ein Verfahren gemäß Anspruch 1.

US-A-4597875 beschreibt die Behandlung von Abwasser der Dinitrotoluolproduktion mit Schwefelsäure, wobei die im Abwasser enthaltenen Di- und Trinitrokresole als Flüssigkeit abgeschieden und durch Verbrennung entsogt werden. Für die Behandlung des Abwassers der Mononitrotoluolproduktion ist dieses Verfahren wenig geeignet, da unter analogen Bedingungen beim Ansäuern des Abwassers der Mononitrotoluolproduktion die enthaltenen Nitrokresole als sehr fein verteilter, fester Niederschlag ausfallen, wobei der Niederschlag sich schlecht von der wässrigen Phase abtrennen lässt. Wegen des anhaftenden Restwassers ist der abgetrennte feste Niederschlag in einem kontinuierlichen Verfahren auch schlecht handhabbar.

US 4,986,917 beschreibt die selektive Rückgewinnung von bestimmten Nebenprodukten, die bei der Nitrierung von aromatischen Verbindungen anfallen. Es wird offenbart, dass durch stufenweises Absenken des pH-Wertes des Waschwassers selektiv die im Abwasser vorhandenen organischen Verunreinigungen, wie 2,4-Dinitrophenol und Pikrinsäure, extrahiert werden können, so dass diese als Reinstoff kommerziell verkauft werden können.

US 4,257,986 offenbart ein Verfahren zur Herstellung von Nitrotoluolen durch Umsetzung von Toluol mit Nitriersäure. Im Anschluss an die Nitrierung wird die Nitriersäure von der nitrierten Verbindung in einem Phasenabscheider getrennt. Die Säure wird zunächst mit einem Oxidationsmittel und/oder Reduktionsmittel behandelt, um salpetrige Säure (HNO₂) zu entfernen. Anschließend wird die säurehaltige Phase mit Toluol in Kontakt gebracht. Dabei reagiert die in der sauren Phase vorhandene Salpetersäure mit Toluol zu Nitrotoluol. Es wird beschrieben, dass das nitrotoluolhaltige Toluol anschließend in der Herstellung von DNT mit verwendet werden kann.

Nitrotoluol wird im technischen Maßstab durch Einwirkung eines Salpeter-Schwefelsäure-Gemisches auf Toluol hergestellt. Hierbei entstehen neben den gewünschten Mononitrotoluolisomeren durch oxidative Einwirkung auf das Toluol auch verschiedene Nitrokresolverbindungen als unerwünschte Nebenprodukte in Anteilen von weniger als ein Prozent. Ihre Entfernung aus dem Reaktionsprodukt ist vor dessen weiterer Verwendung, wie z.B. der Isomerentrennung durch Destillation oder Schmelzkristallisation, unbedingt notwendig.

Zur Entfernung der Nitrokresole aus dem Rohprodukt wird dieses üblicherweise zuerst mit Wasser zur Entfernung von Säurespuren und nachfolgend mit einer basischen Lösung, wie z.B. verdünnter Natronlauge, Sodalösung oder Ammoniaklösung, gewaschen. Dabei gehen die relativ sauren Nitrokresole als Natriumnitrokresolate fast vollständig in die wässrige Phase über. Nach der Phasentrennung erhält man das sogenannte Mononitrotoluol-Abwasser, nachfolgend als MNT-Abwasser bezeichnet.

Um den MNT-Abwasserstrom einer kontinuierlich betriebenen Anlage für die Einleitung in ein als Vorfluter benutztes Gewässer vorzubereiten, wird das MNT-Abwasser einer biologischen Abwasserbehandlung unterzogen. Die im MNT-Abwasser enthaltenen Nitrokresole haben sich als biologisch schwer abbaubar erwiesen.

Daraus ergibt sich die Aufgabe, ein Verfahren zu entwickeln, das die Nitrokresole nahezu vollständig aus dem MNT-Abwasser entfernt und erlaubt, die abgetrennten Nitrokresole in einer einfachen und wirtschaftlich vertretbaren Weise zu beseitigen bzw. einer weiteren Verwendung zuzuführen.

Die Aufgabe wurde erfindungsgemäß dadurch gelöst, dass das alkalische Abwasser der MNT-Herstellung mit Säuren auf einen pH-Wert von höchstens 3 angesäuert wird und die Nitrokresole mit Toluol, o-, m-, p-Nitrotoluol oder deren Gemischen, extrahiert werden.

Gegenstand der Erfindung ist die Herstellung von Dinitrotoluol durch Verwendung der nitrokresolhaltigen Extrakte als Einsatzstoff.

Es wurde ein Verfahren gefunden, bei dem das Ansäuern des alkalischen MNT-Abwassers mit einer Extraktion unter Verwendung eines geeigneten flüssigen Extraktionsmittels kombiniert wird, wobei der erhaltene, nitrokresolhaltige Extrakt der Dinitrotoluolproduktion als Seitenstrom zugeführt werden kann.

Zum Ansäuern des alkalischen MNT-Abwassers werden vorteilhafterweise Säuren mit einem pKₛ-Wert von höchstens 2, vorzugsweise unter 1, eingesetzt. Das sind insbesondere Mineralsäuren, wie beispielsweise Schwefelsäure und Salpetersäure. Besonders bevorzugt werden Schwefelsäure oder Salpetersäure oder deren Gemische eingesetzt.

Die Menge der Säure wird erfindungsgemäß so gewählt, dass das Abwasser nach dem Ansäuern einen pH-Wert von höchstens 3, vorteilhafterweise einen pH-Wert von höchstens 2 aufweist.

Die Extraktion erfolgt unter Verwendung eines geeigneten flüssigen Extraktionsmittels. Im Hinblick auf die Weiterverwendung des nitrokresolhaltigen Extraktes in der Dinitrotoluolproduktion werden als Extraktionsmittel Toluol oder bei Prozesstemperatur der Extraktion flüssige Mononitrotoluole (o-, m-, p-Nitrotoluol) oder deren Isomerengemische oder Gemische aus Toluol und Mononitrotoluolisomeren verwendet.

Das Volumenverhältnis des zu extrahierenden Abwassers zum Extraktionsmittel sollte vorteilhafterweise 1 zu 1 bis 50 zu 1. Vorzugsweise wird gearbeitet bei einem Verhältnis von 2 zu 1 bis 10 zu 1. Besonders gute Ergebnisse werden erzielt bei einem Verhältnis von 2 zu 1 bis 5 zu 1.

Im Hinblick auf die Weiterverwendung des nitrokresolhaltigen Extraktes in der Dinitrotoluolproduktion werden als Extraktionsmittel vorzugsweise Toluol oder bei Prozesstemperatur der Extraktion flüssige Mononitrotoluole (o-, m-, p-Nitrotoluol) oder deren Isomerengemische oder Gemische aus Toluol und Mononitrotoluolisomeren verwendet.

Als Extraktionstemperatur wird vorteilhafterweise etwa die Temperatur gewählt, bei der das MNT-Abwasser aus der vorhergehenden Wäsche anfällt. Der bevorzugte Temperaturbereich liegt zwischen 25 und 60°C, insbesondere zwischen 30 und 55°C.

Die Reihenfolge der Dosierung von Säure und Extraktionsmittel zum Abwasser ist beliebig. Nach der Zugabe beider Komponenten ist ein intensives Vermischen der organischen und wässrigen Phasen vorzunehmen, um den Stoffübergang der Nitrokresole in die organische Phase zu erreichen. Geeignete Apparate hierfür sind beispielsweise Rührkessel, Gegenstromkolonnen oder statische Mischer. Die anschließende Phasentrennung erfolgt vorteilhafterweise in statischen Separatoren durch die natürliche Schwerkraft oder in dynamischen Separatoren (Zentrifugen).

Der nitrokresolhaltige Extrakt enthält dabei vorwiegend Dinitrokresol, aber auch geringe Mengen an Mono- und Trinitrokresolen.

Es ist ein besonderer Vorteil dieses Verfahrens, dass die extrahierten Nitrokresole durch Einschleusen der nitrokresolhaltigen Extrakte in einen Prozess der Herstellung von Dinitrotoluol weiterverwendet werden können. Dabei wird überraschenderweise der größte Teil der extrahierten Nitrokresole abgebaut. Es wäre zu erwarten gewesen, dass durch die Mitverwendung des Extraktes der Gesamtgehalt an Nitrokresolen im DNT-Abwasser deutlich anstiege.

Es ist prinzipiell unerheblich, ob die Extrakte als Teilstrom der Prozessstufe der Mononitrierung des Toluols oder der Prozessstufe der Dinitrierung zugegeben werden. Vorzugsweise wird, insbesondere bei Verwendung von Toluol als Extraktionsmittel, der erhaltene Extrakt der Prozessstufe der Mononitrierung des Toluols zugegeben. Bei Verwendung von Nitrotoluol als Extraktionsmittel ist die Zugabe zur Prozessstufe der Dinitrierung bevorzugt.

Die dem Prozess der Herstellung von Dinitrotoluol durch die Zugabe der Extrakte zugefügte Menge an Nitrokresolen liegt zwischen 0,01 und 1 Gew.-%, insbesondere zwischen 0,05 und 0,5 Gew.%, bezogen auf die Menge des erzeugten Dinitrotoluols. Bei Einhaltung dieser Grenzen wird keine signifikante Verschlechterung der Qualität des Dinitrotoluols und keine wesentliche negative Beeinflussung der Entsorgung des Prozessabwassers der Dinitrotoluolherstellung beobachtet.

Der Vorteil des gefundenen Verfahrens besteht somit in der kostengünstigen Entfernung der Nitrokresole aus dem Abwasser der Mononitrotoluolherstellung und deren einfacher Beseitigung. Vorteilhaft ist weiterhin, dass die Nitrokresole fast quantitativ entfernt werden, kein gesonderter Verfahrensschritt zu deren Entsorgung benötigt wird und dass keine zusätzliche Wärmeenergie zu- oder abgeführt werden muss.

Die Erfindung wird in den nachfolgenden Ausführungsbeispielen näher erläutert, ohne jedoch hierdurch eine entsprechende Eingrenzung vorzunehmen.

### Beispiel 1

### Extraktion von MNT-Abwasser mit Toluol im Verhältnis 10 : 1

In einem Rundkolben mit Rührer und Tropftrichter wurden 1 l alkalisches MNT-Abwasser mit einem Gehalt von 2620 mg/l Dinitrokresolen mit 0,1 l Toluol intensiv vermischt. Um einen pH-Wert von ca. 2 einzustellen, wurden 7 ml 93 %ige Schwefelsäure zugetropft. Anschließend wurde noch 30 Minuten gerührt. Nach Abstellen des Rührers wurde die vollständige Phasentrennung abgewartet. Das so behandelte MNT-Abwasser als wässrige Phase wies noch einen Restgehalt an Dinitrokresolen von 46 mg/l auf.

### Beispiel 2

### Extraktion von MNT-Abwasser mit Mononitrotoluol im Verhältnis 10 : 1

Es wurde wie in Beispiel 1 verfahren, jedoch wurden die 0,1 l Toluol durch 0,1 l eines technisch hergestellten Isomerengemisches der drei Mononitrotoluole ersetzt. Das so behandelte MNT-Abwasser als wässrige Phase wies noch einen Restgehalt an Dintrokresolen von 32 mg/l auf.

### Beispiel 3

### Extraktion von MNT-Abwasser mit Mononitrotoluol im Verhältnis 3 : 1

Es wurde wie in Beispiel 1 verfahren, jedoch wurden die 0,1 l Toluol durch 0,33 l eines technisch hergestellten Isomerengemisches der drei Mononitrotoluole ersetzt. Das so behandelte MNT-Abwasser als wässrige Phase wies noch einen Restgehalt an Dintrokresolen von 8 mg/l auf.

### Beispiel 4

### Nitrierung von Toluol zu Dinitrotoluol unter Zusatz von dinitokresolhaltigem Toluol

In einem 0,5 l-Vierhalskolben mit Rührer, Rückflusskühler, Tropftrichter und Thermometer wurden einem Gemisch aus 65 g Toluol mit 99,8 %iger Reinheit und 4 g dinitrokresolhaltiges Toluol (organische Phase aus Beispiel 1) vorgelegt, wobei im Toluol ca. 120 mg Dinitrokresole enthalten waren. 167 g einer Nitriersäure, bestehend aus 53 Masse-% 96 %iger Schwefelsäure, 40 Masse-% 98 %iger Salpetersäure und 7 Masse-% Wasser, wurden langsam unter Kühlen zugetropft, ohne dass die Temperatur im Reaktionsgemisch 45°C überstieg. Nach Dosierende wurde 1 Stunde bei 35°C nachgerührt. Nach der Phasentrennung bei Raumtemperatur wurden das entstandene Mononitrotoluol als organische Phase in einen 0,5 l-Vierhalskolben mit Rührer, Rückflusskühler, Tropftrichter und Thermometer überführt und 125 g einer Nitriersäure, bestehend aus 71 Masse-% 96 %iger Schwefelsäure und 29 Masse-% 98 %iger Salpetersäure, langsam unter Kühlen zugetropft, ohne dass die Temperatur im Reaktionsgemisch 60°C überstieg. Nach Dosierende wurde 1 Stunde bei 60°C nachgerührt. Nach der Phasentrennung bei 60°C wurde das entstandene Dinitrotoluol als organische Phase zweimal durch intensives Rühren mit 100 ml destilliertem Wasser und anschließender Phasentrennung bei 60°C gewaschen. Zur Entfernung der Dinitrokresole wurde das gewaschene Dinitrotoluol mit 100 ml einer 5 %igen Sodalösung 15 Minuten bei 60°C intensiv verrührt. Nach der Phasentrennung bei 60°C wurde in der Sodalösung (wässrige Phase) ein Dinitrokresolgehalt von 582 mg/l bestimmt.

### Beispiel 5 - Vergleichsbeispiel:

### Nitrierung von Toluol zu Dinitrotoluol ohne Zusatz von dinitokresolhaltigem Toluol

Es wurde wie in Beispiel 4 verfahren, jedoch wurde statt des Gemisches aus 65 g Toluols mit 99,8 %iger Reinheit und 4 g dinitrokresolhaltigen Toluols als Einsatzstoff jetzt 69 g Toluol mit 99,8 %iger Reinheit verwendet. Nach der Phasentrennung bei 60°C wurde in der Sodalösung (wässrige Phase) ein Dinitrokresolgehalt von 572 mg/l bestimmt.

Das Vergleichsbeispiel zeigt, dass durch die Mitverwendung von dinitrokresolhaltigem Toluol im Beispiel 4 keine nennenswerte Erhöhung der Dinitrokresole im Dinitrotoluol entsteht.

## Patentansprüche

1. Verfahren zur Herstellung von Dinitrotoluol durch Nitrierung von Toluol, **dadurch gekennzeichnet, dass** man nitrokresolhaltige Extrakte als Einsatzstoff mitverwendet und die durch Zugabe der Extrakte zugefügte Menge an Nitrokresolen zwischen 0,01 und 1 Gew.-%, bezogen auf die Menge des erzeugten Dinitrotoluols beträgt und dass die nitrokresolhaltigen Extrakte dadurch erhalten werden in dem man alkalisches Abwasser aus der Mononitrotoluolherstellung mit Säuren auf einen pH-Wert von höchstens 3 ansäuert und die Nitrokresole mit einem Extraktionsmittel behandelt werden, wobei Toluol, p-, m-, o-Nitrotoluol oder deren Isomerengemische oder Gemische als Extraktionsmittel für die Extrakte verwendet werden.

2. Verfahren nach Anspruch 1, **dadurch gekennzeichnet, dass** die nitrokresolhaltigen Extrakte als Teilstrom der Prozessstufe der Mononitrierung des Toluols zugegeben werden.

3. Verfahren nach Anspruch 1, **dadurch gekennzeichnet, dass** die nitrokresolhaltigen Extrakte als Teilstrom der Prozessstufe der Dinitrierung zugegeben werden.

4. Verfahren nach mindestens einem der Anspruche 1 bis 4 **dadurch gekennzeichnet, dass** Säuren mit einem pKs-Wert von höchstens 2 eingesetzt werden.

5. Verfahren nach mindestens einem der Anspruche 1 bis 5, **dadurch gekennzeichnet, dass** Mineralsäuren eingesetzt werden.

6. Verfahren nach mindestens einem der Anspruche 1 bis 6, **dadurch gekennzeichnet, dass** das Volumenverhältnis des zu extrahierenden Abwassers zum Extraktionsmittel 1 zu 1 bis 50 zu 1 beträgt.

7. Verwendung von nitrokresolhaltigen Extrakten als Einsatzstoff bei der Herstellung von Dinitrotoluol, **dadurch gekennzeichnet, dass** die durch Zugabe der Extrakte zugefügte Menge an Nitrokresolen zwischen 0,01 und 1 Gew.-%, bezogen auf die Menge des erzeugten Dinitrotoluols, beträgt und dass die nitrokresolhaltigen Extrakte dadurch erhalten werden in dem man alkalisches Abwasser aus der Mononitrotoluolherstellung mit Säuren auf einen pH-Wert von höchstens 3 ansäuert und die Nitrokresole mit einem Extraktionsmittel behandelt, wobei Toluol, p-, m-, o-Nitrotoluol oder deren Isomerengemische oder Gemische als Extraktionsmittel für die Extrakte verwendet werden.

## Claims

1. A process for preparing dinitrotoluene by nitration of toluene, which comprises using nitrocresol-containing extracts as part of the feedstock, the amount of nitrocresols added by addition of the extracts being between 0.01 and 1% by weight, based on the amount of the dinitrotoluene obtained, and the nitrocresol-containing extracts being obtained by acidifying alkaline wastewater from the mononitrotoluene preparation to a pH of at most 3 with acids, and treating the nitrocresols with an extractant, using toluene, p-, m-, o-nitrotoluene or isomer mixtures thereof or mixtures as extractants for the extracts.

2. The process according to claim 1, wherein the nitrocresol-containing extracts are added as a substream to the process stage of mononitration of toluene.

3. The process according to claim 1, wherein the nitrocresol-containing extracts are added as a substream to the process stage of dinitration.

4. The process according to at least one of claims 1 to 3, wherein acids having a pKa value of at most 2 are used.

5. The process according to at least one of claims 1 to 4, wherein mineral acids are used.

6. The process according to at least one of claims 1 to 5, wherein the volume ratio of the wastewater to be extracted to the extractant is from 1:1 to 50:1.

7. The use of nitrocresol-containing extracts as a feedstock in the preparation of dinitrotoluene, wherein the amount of nitrocresols added by addition of the extracts is between 0.01 and 1% by weight, based on the amount of dinitrotoluene obtained, and the nitrocresol-containing extracts are obtained by acidifying alkaline wastewater from the mononitrotoluene preparation to a pH of at most 3 with acids, and treating the nitrocresols with an extractant, using toluene, p-, m-, o-nitrotoluene or isomer mixtures thereof or mixtures as extractants for the extracts.

## Revendications

1. Procédé pour la préparation de dinitrotoluène par nitration de toluène, **caractérisé en ce qu'**on utilise conjointement des extraits contenant du nitrocrésol comme substance de départ et la quantité ajoutée de nitrocrésols par l'addition des extraits est située entre 0,01 et 1% en poids, par rapport à la quantité de dinitrotoluène produit et **en ce que** les extraits contenant du nitrocrésol sont obtenus **en ce qu'**on acidifie des eaux usées alcalines de la préparation du mononitrotoluène par des acides à un pH d'au plus 3 et les nitrocrésols sont traités avec un agent d'extraction, le toluène, le p-nitrotoluène, le m-nitrotoluène, l'o-nitrotoluène ou leurs mélanges d'isomères ou des mélanges étant utilisés comme agent d'extraction pour les extraits.

2. Procédé selon la revendication 1, **caractérisé en ce que** les extraits contenant du nitrocrésol sont ajoutés comme flux partiel aux étapes de procédé de la mononitration du toluène.

3. Procédé selon la revendication 1, **caractérisé en ce que** les extraits contenant du nitrocrésol sont ajoutés comme flux partiel aux étapes de procédé de la dinitration.

4. Procédé selon au moins l'une quelconque des revendications 1 à 3, **caractérisé en ce qu'**on utilise des acides présentant un pKs d'au plus 2.

5. Procédé selon au moins l'une quelconque des revendications 1 à 4, **caractérisé en ce qu'**on utilise des acides minéraux.

6. Procédé selon au moins l'une quelconque des revendications 1 à 5, **caractérisé en ce que** le rapport volumique de l'eau usée à extraire à l'agent d'extraction est de 1:1 à 50:1.

7. Utilisation d'extraits contenant du nitrocrésol comme substance de départ lors de la préparation de dinitrotoluène, **caractérisée en ce que** la quantité ajoutée de nitrocrésols par l'addition des extraits est située entre 0,01 et 1% en poids, par rapport à la quantité de dinitrotoluène produit et **en ce que** les extraits contenant du nitrocrésol sont obtenus **en ce qu'**on acidifie des eaux usées alcalines de la préparation du mononitrotoluène par des acides à un pH d'au plus 3 et on traite les nitrocrésols avec un agent d'extraction, le toluène, le p-nitrotoluène, le m-nitrotoluène, l'o-nitrotoluène ou leurs mélanges d'isomères ou des mélanges étant utilisés comme agent d'extraction pour les extraits.
